# EUROPÄISCHE PATENTANMELDUNG

(11) **EP 3 590 489 A1**
(43) Veröffentlichungstag der Anmeldung: **08.01.2020**
(21) Anmeldenummer: 18182099.4
(22) Anmeldetag: 06.07.2018
(51) Int. Cl.: A61K 6/083, C08F 222/10

(54) **DENTALWERKSTOFFE AUF BASIS VON MONOFUNKTIONELLEN BIPHENYLMETHACRYLATEN**

(71) Anmelder: Ivoclar Vivadent AG, 9494 Schaan (LI)
(72) Erfinder: MOSZNER, Norbert, 9495 Triesen (LI); ANGERMANN, Jörg, 7320 Sargans (CH); FISCHER, Urs Karl, 9320 Arbon (CH)
(74) Vertreter: Uexküll & Stolberg

(57) **Zusammenfassung**

Radikalisch polymerisierbares, monofunktionelles Biphenylmethacrylat gemäß der Formel I 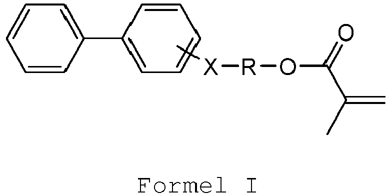 in der R ein linearer oder verzweigter C₁-C₁₄-Alkylrest ist, der durch O oder S unterbrochen sein kann, X entfällt oder -O-, -CO-O- oder -O-CO- ist und die Substitution am Aromaten an Position 2, 3 oder 4 erfolgt.

## Beschreibung

Die vorliegende Erfindung betrifft radikalisch polymerisierbare, thermisch- und/oder lichthärtende Zusammensetzungen auf der Basis von monofunktionellen Biphenylmethacrylaten zur Herstellung von Dentalwerkstoffen, wie z.B. Zementen und Füllungskompositen für Inlays, Onlays, Kronen, Brücken- oder Verblendmaterialien.

Dentalwerkstoffe, die z.B. als Zement oder als direktes Füllungsmaterial eingesetzt werden, enthalten in der Regel eine polymerisierbare organische Matrix und einen oder mehrere Füllstoffe, die meist mit einem polymerisationsfähigen Haftvermittler oberflächenmodifiziert sind. Der Füllstoffgehalt hängt maßgeblich vom gewünschten Verwendungszweck ab und kann bis zu 90 Gew.-% betragen, wobei Befestigungszemente im Vergleich zu Füllungsmaterialien einen geringeren Füllungsgrad aufweisen. Die polymerisierbare organische Matrix enthält gewöhnlich eine Mischung von Monomeren, Initiatorkomponenten, Stabilisatoren und Pigmenten. Dentalwerkstoffe, die eine polymerisierbare Matrix und Füllstoff enthalten, werden als Komposite bezeichnet. Die polymerisierbare Matrix wird auch als Harz bezeichnet.

Polymere Dentalwerkstoffe sind im weitesten Sinne Materialen für eine dentale Anwendung, die auf Polymeren beruhen. Haupteinsatzgebiete von Polymeren im Dentalbereich sind abnehmbare (z.B. Zähne und Prothesenbasismaterialien) und festsitzende (z.B. Verblendmaterialen, Kronen oder Zemente) Prothesen, Füllungsmaterialien (z.B. Füllungskomposite oder Adhäsive) oder Hilfsmaterialien (z.B. Abformmaterialien).

Die polymerisierbare organische Matrix von dentalen Füllungskompositen und Kompositzementen besteht meistens aus einer Mischung von Harzmonomeren, Initiator-Komponenten und Stabilisatoren. Als Harze werden meistens Mischungen von Dimethacrylaten eingesetzt.

Verbreitete Beispiele hierfür sind die hochviskosen Dimethacrylate 2,2-Bis[4-(2-hydroxy-3-methacryloyloxypropyl)-phenyl]propan (Bis-GMA) und 1,6-Bis-[2-methacryloyloxyethoxy-carbonylamino]-2,2,4-trimethylhexan (UDMA) und die als Verdünnermonomere genutzten niedrigerviskosen Dimethacrylate Decandiol-1,10-dimethacrylat (D₃MA) und Triethylenglycoldimethacrylat (TEGDMA). Dimethacrylate bewirken bei der Polymerisation eine dreidimensionale Vernetzung der sich bildenden Polymerketten und ergeben damit eine hohe mechanische Stabilität.

Die Werkstoffe enthalten gewöhnlich auch einen Initiator für die radikalische Polymerisation, wobei lichthärtende Werkstoffe, die einen Photoinitiator enthalten, in der zahnärztlichen Füllungstherapie heutzutage eine dominante Stellung einnehmen.

Ein Nachteil von lichthärtenden Werkstoffen ist, dass insbesondere das Legen großer Füllungen mit einem erheblichen Aufwand verbunden ist, weil das zur Härtung erforderliche Licht nur bis zu einer begrenzten Tiefe in die Werkstoffe eindringen kann. Bei der sogenannten Inkrementtechnik wird die Füllung daher schichtweise aus dem Kompositwerkstoff aufgebaut, wobei die Schichten eine Dicke von jeweils etwa 2 mm haben und einzeln gehärtet werden.

Neuerdings finden sogenannte "Bulk-Fill"-Komposite, die Schichtdicken von 4 bis 5 mm erlauben, aufgrund der möglichen Zeitersparnis großes Interesse. Voraussetzung für die klinische Eignung dieser Werkstoffe ist eine große Durchhärtungstiefe.

Die Durchhärtungstiefe ist sowohl von Prozessparametern als auch von den Werkstoffeigenschaften abhängig. So besteht z.B. zwischen der Durchhärtungstiefe und der Intensität des eingestrahlten Lichtes bzw. der Belichtungszeit ein logarithmischer Zusammenhang. Weiterhin korreliert die Durchhärtungstiefe mit der Transparenz und Transluzenz der Werkstoffe. Üblicherweise spricht man bei füllstoffhaltigen Werkstoffen von Transluzenz statt von Transparenz (Durchsichtigkeit), weil sie Licht zwar durchlassen, in der Regel aber nicht transparent sind. Als transluzent werden in der Technik Körper mit einer Lichtdurchlässigkeit von kleiner als 90% bezeichnet.

Die Lichtdurchlässigkeit von Polymeren ist im Idealfall (keine Absorption und Brechung) von deren Brechungsindex n abhängig und variiert zwischen 98,4 % (n = 1,29) und 92,8 % (n = 1,73) (vgl. H.-G. Elias, Makromoleküle - Anwendungen von Polymeren, Bd. 4. 6. Auflage, Wiley-VCH, Weinheim 2003, 517). Diese Idealwerte werden jedoch nur selten erreicht, weil das Licht gestreut und absorbiert wird.

Die Lichtdurchlässigkeit von Kompositen wird u.a. durch die Brechungsindices der Harzmatrix und der Füllstoffe, durch die Größe der Füllstoffpartikel (Streuung) sowie die Art und Konzentration zugesetzter Farbmittel (Absorption) beeinflusst. Der Brechungsindex besonders häufig eingesetzter Dentalmonomere nimmt in folgender Reihenfolge ab: Bis-GMA (1,5512), ethoxyliertes Bis-GMA (SR-348c) (1,5393), UDMA (1,4850), TEDGMA (1,4610) und D₃MA (1,4600). Der Brechungsindex der als Füllstoff häufig verwendeten röntgenopaken Gläser liegt im Bereich von 1,523-1,550 und der Brechungsindex von Zahnschmelz bei 1,655.

Bei Kompositen kann eine hohe Lichtdurchlässigkeit und damit eine gute Durchhärtungstiefe dadurch erreicht werden, dass eine organische Matrix und Füllstoffe mit übereinstimmenden Brechungsindices verwendet werden. Aromatische Methacrylate haben häufig hohe Brechungsindices, die annähernd den Brechungsindices röntgenopaker Gläser entsprechen. Andererseits weisen solche Monomere oft eine hohe Viskosität auf und lassen sich erst nach Zugabe von niedrigviskosen Monomeren verarbeiten und mit Füllstoffen mischen. Die im Dentalbereich üblicherweise eingesetzten Verdünnermonomere wie TEGDMA oder D₃MA haben aber nur relative niedrige Brechungsindices, meist deutlich unter 1,50, so dass ihre Zugabe eine Verringerung des Brechungsindex der Harzmischung und damit eine Beeinträchtigung der Lichtdurchlässigkeit bewirkt. Außerdem zeigen diese Verdünnermonomere einen relativ hohen Polymerisationsschrumpf ΔVₚ, z.B. von 14,5 Vol.-% (TEGDMA) oder 10,3 Vol.-% (D₃MA).

Im Vergleich zu Dimethacrylaten finden niedrigviskose monofunktionelle Methacrylate in direkten Füllungskompositen oder Zementen kaum Anwendung. Als nachteilig gilt, dass monofunktionelle Methacrylate im Vergleich zu Dimethacrylaten eine geringere Photopolymerisationsreaktivität besitzen und in Kombination mit Vernetzern zu einer Verringerung der Netzwerkdichte und damit zu einer Verschlechterung der mechanischen Eigenschaften führen können. Darüber hinaus können Polymerisate von Monomethacrylaten bei unvollständiger Polymerisation einen hohen Anteil an in einem wässrigen Milieu wie der Mundhöhle herauswaschbarem Restmonomer enthalten, was toxikologisch bedenklich sein kann. Dementgegen sind Dimethacrylate selbst im Falle der Polymerisation von nur einer Methacrylatgruppe noch an das gebildete Polymernetzwerk gebunden.

Der Erfindung liegt die Aufgabe zugrunde, thermisch- und/oder lichthärtende Werkstoffe zur Verfügung zu stellen, die sich insbesondere für dentale Zwecke eignen und die eine hohe Durchhärtungstiefe, eine relativ niedrige Viskosität, einen geringen Polymerisationsschrumpf, eine hohe Polymerisationsreaktivität und eine geringe Wasserlöslichkeit aufweisen.

Diese Aufgabe wird erfindungsgemäß durch Werkstoffe gelöst, die mindestens ein radikalisch polymerisierbares, monofunktionelles Biphenylmethacrylat gemäß der allgemeinen Formel I enthalten, 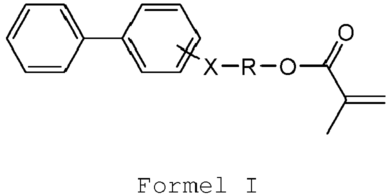 in der
- R: ein linearer oder verzweigter C₁-C₁₄-Alkylrest ist, der durch 0 oder S unterbrochen sein kann,
- X: entfällt oder -O-, -CO-O- oder -O-CO- ist
und die Substitution am Aromaten an Position 2, 3 oder 4 erfolgt.

Die Position 2 entspricht einer Substitution in ortho-Stellung, die Position 3 entspricht einer Substitution in meta-Stellung und die Position 4 entspricht einer Substitution in para-Stellung, jeweils im Verhältnis zur Aryl-Aryl-Bindung von Biphenyl.

Bevorzugt sind Verbindungen, in denen
- R: ein verzweigter oder vorzugsweise linearer C₁-C₈-Alkylrest, insbesondere ein C₂-C₆-Alkylrest, ist, der durch 0 oder S unterbrochen sein kann,
- X: O ist
und die Substitution an Position 2 erfolgt.

Weiterhin bevorzugt sind Verbindungen, in denen
- R: ein verzweigter oder vorzugsweise linearer C₁-C₈-Alkylrest, insbesondere ein C₂-C₆-Alkylrest, ist, der durch 0 oder S unterbrochen sein kann,
- X: O ist
und die Substitution an Position 3 erfolgt.

Des Weiteren bevorzugt sind Verbindungen, in denen
- R: ein verzweigter oder vorzugsweise linearer C₁-C₈-Alkylrest, insbesondere ein C₂-C₆-Alkylrest, ist, der durch O oder S unterbrochen sein kann,
- X: -O-CO- oder -CO-O- ist
und die Substitution an Position 2 erfolgt.

Besonders bevorzugt sind Verbindungen, in denen
- R: ein linearer C₂- oder C₄-Alkylrest ist,
- X: O ist
und die Substitution an Position 2 erfolgt.

Die Formel I erstreckt sich nur auf solche Verbindungen, die mit der chemischen Valenzlehre vereinbar sind. Der Hinweis, dass ein Rest durch ein oder mehrere O-Atome unterbrochen ist, ist so zu verstehen, dass diese Atome jeweils in die Kohlenstoffkette des Restes eingeschoben werden. Diese Atome sind damit beidseitig durch C-Atome begrenzt und können nicht endständig sein. C₁-Reste können nicht unterbrochen sein.

Die erfindungsgemäßen monofunktionellen Biphenylmethacrylate der allgemeinen Formel I sind auf an sich bekannte Weise zugänglich und lassen sich nach bekannten Synthesemethoden herstellen. Auf einer möglichen Syntheseroute werden hydroxyalkylierte Biphenylderivate (X = O) durch Veretherung von Hydroxybiphenyl mit α,ω-Hydroxyalkylenchloriden hergestellt. Anschließend werden sie durch Umsetzung mit Methacrylsäure oder Methacrylsäureanhydrid in die erfindungsgemäßen Biphenylmethacrylate der allgemeinen Formel I überführt:

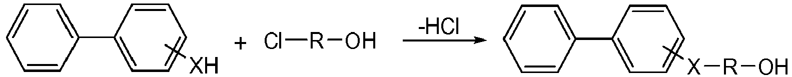

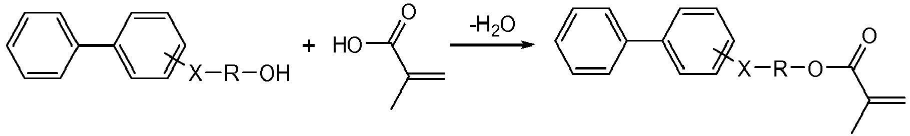

Vorzugsweise lässt sich das besonders bevorzugte 2-(2-Biphenyloxy)-ethylmethacrylat (BPOEMA) aus dem kommerziell zugänglichen 2-Hydroxybiphenyl herstellen. Dabei erfolgt zunächst die Etherbildung durch Umsetzung mit überschüssigem 2-Chlorethanol in Gegenwart von Natriumhydroxid, Kaliumiodid und Benzyltriethylammoniumchlorid bei 60 °C, wobei sich das entsprechende 2-(2-Biphenyl)-oxyethanol bildet, dessen Veresterung mit Methacrylsäureanhydrid bei zunächst 0 °C, dann 20 °C zu 2-(2-Biphenyloxy)-ethylmethacrylat führt.

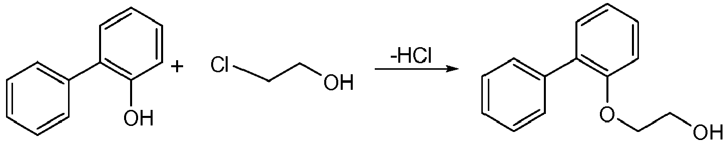

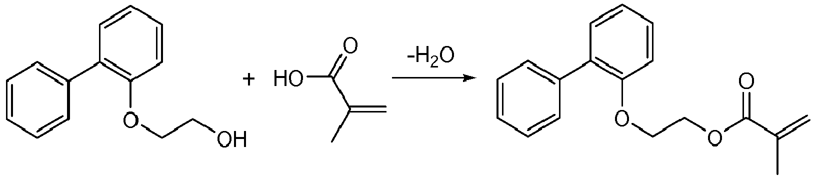

Bevorzugte monofunktionelle Biphenylmethacrylate der allgemeinen Formel I sind:

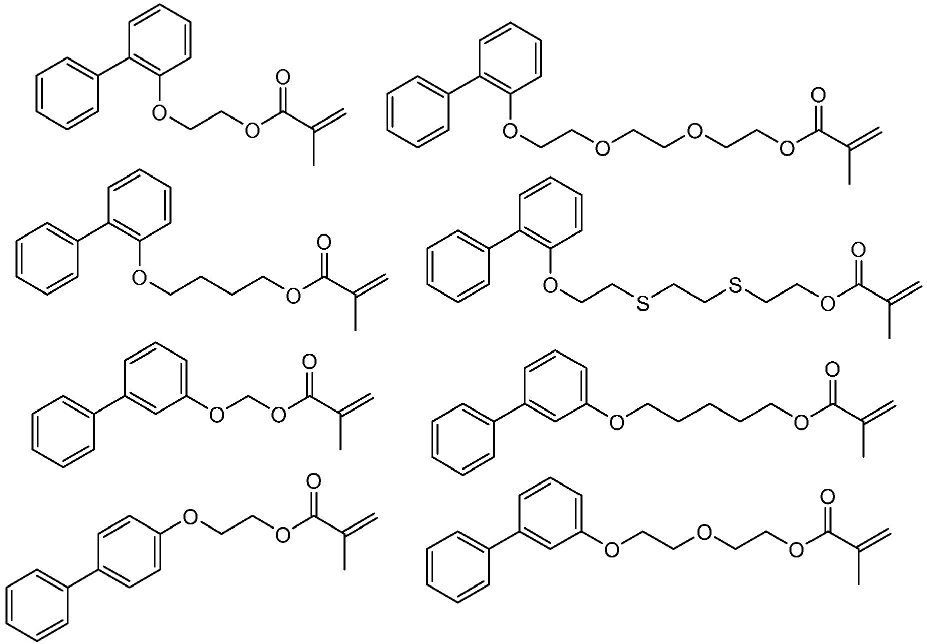

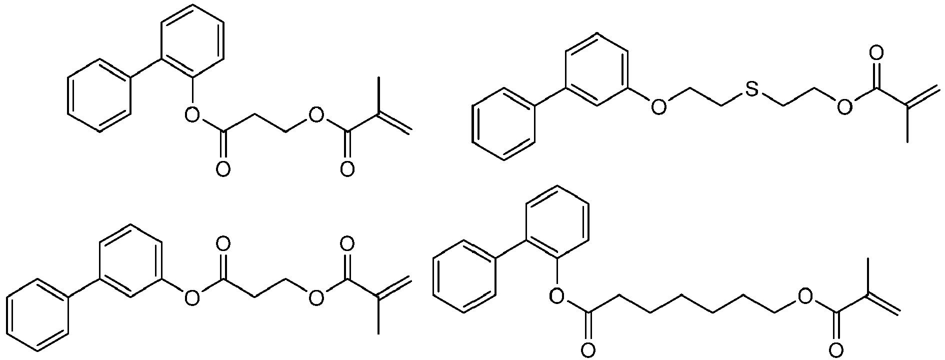

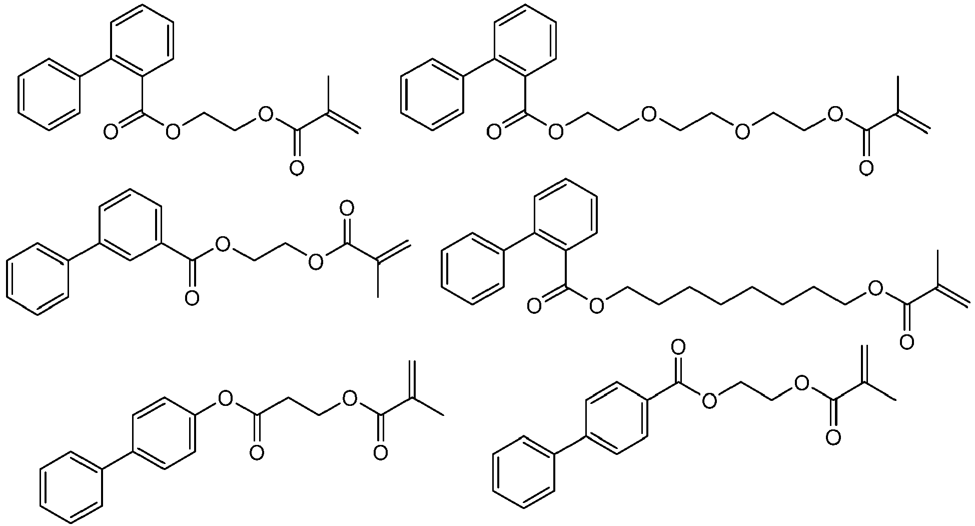

Besonders bevorzugte monofunktionelle Biphenylmethacrylate der allgemeinen Formel I sind:

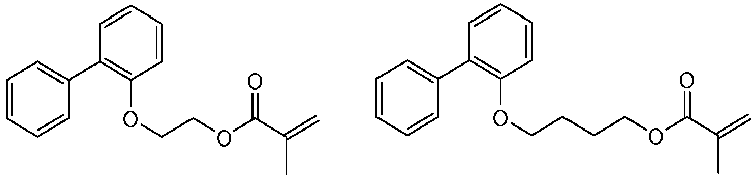

Die Monomere der Formel I zeichnen sich durch eine geringe Viskosität aus. Sie haben vorzugsweise eine Viskosität kleiner als 0,8 Pa·s, bevorzugt kleiner als 0,4 Pa·s, besonders bevorzugt kleiner als 0,2 Pa·s, wobei die Viskosität mit einem Rotationsviskosimeter, vorzugsweise einem MCR 302 Rheometer (Anton Paar GmbH, Österreich) mit einem Platte-Platte-Messsystem, bei einer Temperatur von 23 °C bestimmt wird. Hierzu wird die Probe vorher für 3 Minuten temperiert und die Schergeschwindigkeit zwischen 0,1 und 100 s⁻¹ gemessen. Aus dem für die Bewegung notwendigen Drehmoment werden die Schubspannung und die Viskosität berechnet.

Die erfindungsgemäßen monofunktionellen Biphenylmethacrylate der Formel I werden allein oder bevorzugt in Mischung mit mindestens einem weiteren radikalisch polymerisierbaren Monomer zur Herstellung von Dentalwerkstoffen, wie z.B. Kompositen oder Materialien zur Herstellung dentaler Prothesen oder von Zähnen, eingesetzt. Besonders bevorzugt sind Werkstoffe, die als radikalisch polymerisierbares Monomer mindestens ein mono- oder multifunktionelles (Meth)acrylat enthalten. Unter monofunktionellen (Meth)acrylaten werden Verbindungen mit einer, unter multifunktionellen (Meth)acrylaten Verbindungen mit zwei oder mehr, vorzugsweise 2 bis 4 radikalisch polymerisierbaren Gruppen verstanden. Gemäß einer ganz besonders bevorzugten Ausführungsform enthalten die erfindungsgemäßen Zusammensetzungen mindestens ein Dimethacrylat.

Bevorzugte multifunktionelle Methacrylate sind Bisphenol-A-dimethacrylat, wie z.B. Bis-GMA (ein Additionsprodukt aus Methacrylsäure und Bisphenol-A-diglycidylether), ethoxy- oder propoxyliertes Bisphenol-A-dimethacrylat, wie z.B. 2-[4-(2-Methacryloyloxyethoxyethoxy)phenyl]-2-[4-(2-methacryloyloxy-ethoxy)phenyl]-propan) (SR-348c, Firma Sartomer,; enthält 3 Ethoxygruppen) oder 2,2-Bis[4-(2-methacryloyloxypropoxy)phenyl]propan, 1,6-Bis-[2-methacryloyloxyethoxycarbonylamino]-2,2,4-trimethylhexan (UDMA; ein Additionsprodukt aus 2-Hydroxyethylmethacrylat und 2,2,4-Trimethylhexamethylen-1,6-diisocyanat), Di-, Tri- oder Tetraethylenglycoldimethacrylat, Trimethylolpropantrimethacrylat, Pentaerythrittetramethacrylat, sowie Glycerindi- und -trimethacrylat, 1,4-Butandioldimethacrylat, 1,10-Decandioldimethacrylat (D₃MA) oder 1,12-Dodecandioldimethacrylat und Mischungen davon. Besonders bevorzugte Monomere sind Bis-GMA, UDMA, D₃MA, Triethylenglycoldimethacrylat (TEGDMA), SR-348c und Mischungen davon. Ganz besonders bevorzugt sind Dentalwerkstoffe, die als radikalisch polymerisierbares Monomer Bis-GMA, UDMA, TEGDMA, D₃MA oder eine Mischung davon enthalten.

Die monofunktionellen Biphenylmethacrylate der Formel I zeichnen sich außerdem durch einen geringen Polymerisationsschrumpf aus. Dieser liegt bevorzugt unter 9,0 Vol.-%, besonders bevorzugt unter 8,5 Vol.-%, ganz besonders bevorzugt unter 8,0 Vol.-%, was für dentale Anwendungen ein wesentlicher Vorteil ist.

Zur Herstellung einer polymerisierbaren Matrix und von Kompositen sind erfindungsgemäß Monomere und Monomermischungen bevorzugt, die einen Brechungsindex haben, der dem des verwendeten Füllstoffs nahekommt und damit eine hohe Transluzenz erwarten lässt. Dentale Füllstoffe haben meist einen Brechungsindex von etwa 1,523 bis 1,550. Das weit verbreitete Bis-GMA weist diesbezüglich zwar einen sehr vorteilhaften Brechungsindex auf (n_{D} = 1,5512), seine hohe Viskosität erschwert jedoch das Einarbeiten von Füllstoffen und schränkt die maximale Füllstoffmenge stark ein. Übliche Verdünnermonomere haben meist einen Brechungsindex von deutlich unter 1,50 und bewirken daher neben der angestrebten Verringerung der Viskosität auch eine unerwünschte Absenkung des Brechungsindexes der Monomermischung. Die erfindungsgemäßen monofunktionellen Biphenylmethacrylate der allgemeinen Formel I zeichnen sich demgegenüber durch eine geringe Viskosität und einen hohen Brechungsindex aus. Sie ermöglichen daher eine Einstellung der Viskosität der Monomermischung, ohne den Brechungsindex zu verringern, bzw. eine Einstellung des Brechungsindexes ohne die Viskosität zu erhöhen.

Erfindungsgemäß bevorzugte Verbindungen der Formel I haben einen Brechungsindex von mehr als 1,50, bevorzugt von 1,53 bis 1,65 und besonders bevorzugt von 1,54 bis 1,60.

Der Brechungsindex ist eine Stoffkonstante, die von der Wellenlänge des verwendeten Lichts, von der Temperatur, vom Druck und der Reinheit des Stoffes abhängt. Wenn nicht anders angegeben wird hier unter dem Brechungsindex der bei 20 °C mit dem Licht der gelben Na-D-Linie (λ = 589 nm) gemessene Brechungsindex verstanden (n_{D}²⁰ oder kurz n_{D}). Die Bestimmung des Brechungsindex flüssiger Monomere und Monomermischungen kann mit einem handelsüblichen Abbe-Refraktometer erfolgen.

Weiterhin enthalten die erfindungsgemäßen Werkstoffe vorzugsweise mindestens einen Photoinitiator oder thermischen Initiator für die radikalische Polymerisation, besonders bevorzugt einen Photoinitiator, insbesondere einen Photoinitiator, der in einem Wellenlängenbereich von 400 bis 500 nm aktiv ist.

Bevorzugte Photoinitiatoren sind Benzophenon, Benzoin, α-Diketone, wie 9,10-Phenanthrenchinon, 1-Phenyl-propan-1,2-dion, Diacetyl oder 4,4'-Dichlorbenzil oder deren Derivate, besonders bevorzugt Campherchinon (CC) und 2,2-Dimethoxy-2-phenyl-acetophenon, und Mischungen davon.

Die Photoinitiatoren werden vorzugsweise in Kombination mit Beschleunigern eingesetzt. Als Beschleuniger eignen sich besonders tertiäre Amine, wie z.B. tertiäre aromatische Amine, insbesondere N,N-Dialkyl-aniline, -p-toluidine oder -3,5-xylidine, p-N,N-Dialkylamino-phenylethanol, -benzoesäurederivate, -benzaldehyd, -phenylessigsäureester und -phenylpropionsäureester. Am meisten bevorzugt sind α-Diketone in Kombination mit Aminen als Reduktionsmittel, wie z.B. 4-(Dimethylamino)-benzoesäureethylester (EDMAB), N,N-Dimethylaminoethylmethacrylat, N,N-Dimethyl-sym.-xylidin oder Triethanolamin. Ganz besonders bevorzugt ist die Kombination von CC und EDMAB.

Besonders bevorzugte Photoinitiatoren sind Norrish-Typ-I-Photoinitiatoren, bevorzugt Acyl- oder Bisacylphosphinoxide, und besonders bevorzugt Monoacyltrialkyl- bzw. Diacyldialkylgermanium- und Tetraacylgermanium-Verbindungen, wie z.B. Benzoyltrimethylgerman, Dibenzoyldiethylgerman, Bis(4-methoxybenzoyl)diethylgerman (Ivocerin®), Tetrabenzoylgerman oder Tetrakis(o-methylbenzoyl)german. Acyl- und Bisacylgermanium-Verbindungen haben den Vorteil, dass sie sich nach der Bestrahlung entfärben (Bleaching Effekt) und so die Transparenz der ausgehärteten Werkstoffe nicht beeinträchtigen. Außerdem handelt es sich um monomolekulare Photoinitiatoren, d.h. sie benötigen keinen Beschleuniger, um ihre volle Aktivität zu erreichen.

Des Weiteren bevorzugt sind Mischungen der verschiedenen oben genannten Photoinitiatoren, wie z.B. eine Mischung von Bis(4-methoxybenzoyl)diethylgerman oder Tetrakis(o-methylbenzoyl)-german mit Campherchinon und 4-Dimethylaminobenzoesäureethylester.

Die erfindungsgemäßen Werkstoffe können zusätzlich einen oder mehrere weitere Initiatoren für die radikalische Polymerisation enthalten, bevorzugt Azoverbindungen, wie z.B. 2,2'-Azobis-(isobutyronitril) (AIBN) oder Azobis-(4-cyanovaleriansäure), Peroxide, wie z.B. Dibenzoylperoxid, Dilauroylperoxid, *tert-*Butylperoctoat, tert-Butylperbenzoat oder Di-(tert-butyl)-peroxid, oder Redox-Initiatorkombinationen, wie z.B. Kombinationen von Benzoylperoxid mit Aminen, wie z.B. N,N-Dimethyl-p-toluidin N,N-Dihydroxyethyl-p-toluidin, p-Dimethylaminobenzoesäureethylester oder N,N-Dimethyl-sym.-xylidin, Kombinationen von anorganischen Peroxiden, wie z.B. Kalium- und Ammoniumperoxodisulfat, mit Reduktionsmitteln, wie z.B. Sulfit, Hydrogensulfit, Thiosulfat, Sulfinsäuren, Aminen, Endiolen oder Fe-(II)-Salzen, Redoxsysteme bestehend aus organischen Peroxiden bzw. Hydroperoxiden und Reduktionsmitteln, wie z.B. Ascorbinsäure, Barbituraten, Thioharnstoffen oder Sulfinsäuren.

Weiterhin lassen sich Verbindungen von Übergangsmetallen, die mindestens zwei stabile Wertigkeitsstufen aufweisen, als Übergangsmetall-Redoxkatalysatoren einsetzen. Das sind vor allem Verbindungen der Elemente Kupfer, Eisen, Vanadium, Nickel oder Kobalt, wobei Kupferverbindungen besonders bevorzugt sind und diese bevorzugt als gut organolösliche Verbindungen, wie z.B. als Acetylacetonat, Naphthenat oder 2-Ethyl-hexanoat eingesetzt werden.

Die erfindungsgemäßen Dentalwerkstoffe können vorzugsweise anorganische Füllstoffe enthalten, wobei partikuläre Füllstoffe bevorzugt sind. Bevorzugte anorganische partikuläre Füllstoffe sind Oxide, wie SiO₂, ZrO₂ und TiO₂ oder Mischoxide aus SiO₂, ZrO₂, ZnO und/oder TiO₂ mit einer Partikelgröße von 0,010 bis 15 µm, nanopartikuläre oder mikrofeine Füllstoffe mit einer Partikelgröße von 10 bis 300 nm, wie pyrogene Kieselsäure oder Fällungskieselsäure, sowie Glaspulver, wie Quarz-, Glaskeramik- oder röntgenopake Glaspulver, vorzugsweise Barium- oder Strontiumaluminiumsilikatgläser, mit einer Partikelgröße von 0,01 bis 15 µm, vorzugweise von 0,2 bis 1,5 µm, und röntgenopake Füllstoffe, wie Ytterbiumtrifluorid, Tantal(V)-oxid, Bariumsulfat oder Mischoxide von SiO₂ mit Ytterbium(III)-oxid oder Tantal(V)-oxid, mit einer Partikelgröße von 0,2 bis 5 µm. Besonders bevorzugte Füllstoffe sind pyrogene Kieselsäure, Ytterbiumtrifluorid, SiO₂-ZrO₂-Mischoxide und Bariumaluminiumsilikatgläser, wie z.B. das Glas GM 27884 der Firma Schott, vorzugsweise Bariumaluminiumsilikatgläser, die eine Mischung aus BaO, Al₂O₃, SiO₂, und B₂O₃ enthalten, besonders bevorzugt 25 Gew.-% BaO, 10 Gew.-% Al₂O₃, 55 Gew.-% SiO₂ und 10 Gew.-% B₂O₃. Weiterhin können die erfindungsgemäßen Dentalwerkstoffe faserförmige Füllstoffe, Nanofasern, Whiskers oder Mischungen davon enthalten.

Wenn nicht anders angegeben, handelt es sich bei allen Partikelgrößen um gewichtsmittlere Partikelgrößen, bei denen die Partikelgrößenbestimmung mittels statischer Lichtstreuung erfolgte, vorzugsweise mit einem statischen Laserstreuungs-Partikelgrößen-Analysator LA-960 (Horiba, Japan). Hierzu wurden als Lichtquellen eine Laser-Diode mit einer Wellenlänge von 655 nm und eine LED mit einer Wellenlänge von 405 nm in einem Messbereich von 0,1 bis 1000 µm verwendet. Dieser Einsatz von zwei Lichtquellen mit unterschiedlichen Wellenlängen in Verbindung mit der gerätespezifischen Anordnung der Vorwärts-, Seitwärts- und Rückwärtsdetektoren ermöglicht die Vermessung der gesamten Partikelgrößenverteilung einer Probe in nur einem Messungsdurchgang, wobei die Messung als Nassmessung durchgeführt wurde. Hierzu wurde eine 0,1 bis 0,5%ige wässrige Dispersion des Füllstoffs hergestellt und deren Streulicht in einer Durchflusszelle gemessen. Die Streulichtanalyse zur Berechnung von Partikelgröße und Partikelgrößenverteilung erfolgte gemäß der Mie-Theorie nach DIN/ISO 13320.

Die Lichtstreuung nimmt mit abnehmender Partikelgröße ab, jedoch haben Füllstoffe mit geringer Partikelgröße eine größere Verdickungswirkung. Die Füllstoffe werden nach der Partikelgröße unterteilt in Makrofüller und Mikrofüller. Makrofüller werden z.B. durch Mahlen von Quarz, röntgenopaken Gläsern, Borosilikaten oder von Keramik gewonnen, sind rein anorganischer Natur und bestehen meist aus splitterförmigen Teilen. Bevorzugt sind Makrofüller mit einer mittleren Partikelgröße von 0,2 bis 15 µm. Als Mikrofüller werden vorzugsweise pyrogenes SiO₂ oder Fällungskieselsäure eingesetzt oder auch Mischoxide, z.B. SiO₂-ZrO₂, die durch hydrolytische Cokondensation von Metallalkoxiden zugänglich sind. Die Mikrofüller weisen vorzugsweise eine mittlere Partikelgröße von 5 bis 100 nm auf.

Die Füllstoffe sind vorzugsweise oberflächenmodifiziert, besonders bevorzugt durch Silanisierung, ganz besonders bevorzugt durch radikalisch polymerisierbare Silane, insbesondere mit 3-Methacryloyloxypropyltrimethoxysilan. Die Silanisierung hat keinen messbaren Einfluss auf den Brechungsindex des Füllstoffs. Zur Oberflächenmodifizierung von nicht-silikatischen Füllstoffen, z.B. von ZrO₂ oder TiO₂, können auch funktionalisierte saure Phosphate, wie z.B. 10-Methacryloyloxydecyldihydrogenphosphat eingesetzt werden.

Die erfindungsgemäßen Zusammensetzungen können vorteilhaft weitere Additive enthalten, vor allem Stabilisatoren, Farbmittel, antibakterielle Wirkstoffe, fluoridionenabgebende Additive, optische Aufheller, Weichmacher oder UV-Absorber.

Dabei enthalten die Werkstoffe auf der Basis der erfindungsgemässen Biphenylmethacrylate der Formel I und ihrer Mischungen mit multifunktionellen Methacrylaten vorzugsweise die folgenden Komponenten:
a) 0,5 bis 60 Gew.-%, bevorzugt 1 bis 50 Gew.-% und besonders bevorzugt 3 bis 30 Gew.-% mindestens eines monofunktionellen Biphenylmethacrylats der Formel I,
b) 0,01 bis 5 Gew.-%, besonders bevorzugt 0,1 bis 3,0 Gew.-% mindestens eines Initiators für die radikalische Polymerisation,
c) 5 bis 70 Gew.-%, bevorzugt 10 bis 60 Gew.-% und besonders bevorzugt 10 bis 50 Gew.-% mindestens eines weiteren radikalisch polymerisierbaren Monomers und
d) 0 bis 80 Gew.-%, bevorzugt 10 bis 70 Gew.-% und besonders bevorzugt 50 bis 70 Gew.-% mindestens eines Füllstoffs.

Der Füllungsgrad richtet sich nach dem gewünschten Anwendungszweck des Werkstoffs. Kompositzemente haben vorzugsweise einen Füllstoffgehalt von 10 bis 70 Gew.-% und Füllungskomposite einen Füllstoffgehalt von 50 bis 80 Gew.-%.

Alle Mengenangaben (Gew.-%) hierin beziehen sich auf die Gesamtmasse des Dentalwerkstoffs, wenn nicht anders angegeben.

Besonders bevorzugt sind Dentalwerkstoffe, die aus den genannten Stoffen bestehen. Weiterhin sind solche Werkstoffe bevorzugt, bei denen die einzelnen Komponenten jeweils aus den oben genannten bevorzugten und besonders bevorzugten Stoffen ausgewählt sind.

Ganz besonders bevorzugt sind Dentalwerkstoffe, die als radikalisch polymerisierbares Monomer (c) Bis-GMA, UDMA, D₃MA, TEGDMA, SR-348c oder eine Mischung davon enthalten. Außerdem bevorzugt sind Dentalwerkstoffe, die als Füllstoff (d) pyrogene Kieselsäure, Ytterbiumfluorid, SiO₂-ZrO₂-Mischoxide, Bariumaluminiumsilikatgläser, wie z.B. das Glas GM 27884 der Firma Schott, vorzugsweise Bariumaluminiumsilikatgläser, die eine Mischung aus BaO, Al₂O₃, SiO₂, und B₂O₃ enthalten, besonders bevorzugt 25 Gew.-% BaO, 10 Gew.-% Al₂O₃, 55 Gew.-% SiO₂ und 10 Gew.-% B₂O₃, oder eine Mischung davon enthalten. Dentalwerkstoffe, die als Monomer (c) mindestens eines der genannten Monomere und als Füllstoff (d) mindestens einen der genannten Füllstoffe enthalten, sind besonders vorteilhaft.

Es wurde überraschender Weise gefunden, dass die erfindungsgemäßen Werkstoffe, die monofunktionelle Biphenylmethacrylate der Formel I enthalten, vergleichbare mechanische Eigenschaften wie Dentalwerkstoffe aufweisen, die lediglich Dimethacrylate und keine monofunktionellen Methacrylate enthalten. Insbesondere weisen sie nach der Polymerisation eine vergleichbare Biegefestigkeit und ein vergleichbares Biege-E-Modul auf, wie sie gemäß der ISO-Norm ISO-4049 (Dentistry - Polymer-based filing, restorative and luting materials) bestimmt werden. Die erfindungsgemäßen Werkstoffe weisen nach der Polymerisation bevorzugt eine Biegefestigkeit von 50 MPa bis 200 MPa, besonders bevorzugt von 60 MPa bis 180 MPa und ganz besonders bevorzugt von 70 MPa bis 170 MPa und ein Biege-E-Modul von 2,00 GPa bis 15,00 GPa, besonders bevorzugt 3,00 GPa bis 12,00 GPa und ganz besonders bevorzugt von 3,00 GPa bis 10,00 GPa auf.

Die erfindungsgemäßen Werkstoffe eigenen sich besonders als Dentalwerkstoffe, insbesondere als dentale Zemente, Füllungskomposite und Verblendmaterialien sowie als Materialien zur Herstellung von Prothesen, künstlichen Zähnen, Inlays, Onlays, Kronen und Brücken. Die Dentalwerkstoffe eignen sich primär zur intraoralen Anwendung durch den Zahnarzt zur Restauration geschädigter Zähne, d.h. zur therapeutischen Anwendung, z.B. als dentale Zemente, Füllungskomposite und Verblendmaterialien. Sie können aber auch extraoral eingesetzt werden, beispielweise bei der Herstellung oder Reparatur von Dentalrestaurationen, wie Prothesen, künstlichen Zähnen, Inlays, Onlays, Kronen und Brücken.

Die erfindungsgemäßen Werkstoffe eigenen sich außerdem zur Herstellung von Formkörpern für dentale aber auch für nicht dentale Zwecke, die z.B. mittels Gießen, Pressen und insbesondere durch generative Verfahren wie den 3D-Druck hergestellt werden können.

Außerdem betrifft die Erfindung die Verwendung von Biphenylmethacrylaten der Formel I zur Herstellung von Dentalwerkstoffen.

Die Erfindung wird im Folgenden anhand von Beispielen und Figuren näher erläutert.

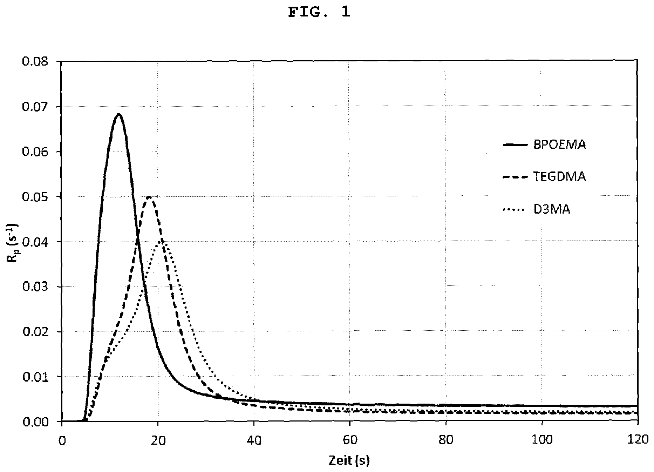

Fig. 1 zeigt einen Vergleich der Polymerisationsreaktivität des erfindungsgemäßen Monomers BPOEMA mit D₃MA und TEGDMA bei 30 °C Reaktionstemperatur und 0,5 mol-% Ivocerin® als Photoinitiator. Die Ergebnisse in Figur 1 belegen eine deutlich höhere Photopolymerisationsreaktivität von BPOEMA mit einer maximalen Polymerisationsgeschwindigkeit Rₚₘₐₓ von 0,068 s⁻¹ im Vergleich zu 0,040 s⁻¹ für D₃MA und 0,050 s⁻¹ für TEGDMA.

### Ausführungsbeispiele

### Beispiel 1: Synthese von 2-(2-Biphenyloxy)-ethylmethacrylat (BPOEMA)

### 1. Stufe: 2-(2-Biphenyl)-oxyethanol

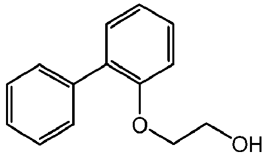

In einem Doppelmantelreaktor wurden zu einer Lösung von 0,90 kg (22,5 mol) Natriumhydroxid in 15,0 kg Wasser 2,55 kg (15,0 mol) 2-Hydroxybiphenyl, 0,12 kg (0,75 mol) Kaliumiodid und 0,17 kg (0,75 mol) Benzyltriethylammoniumchlorid zugegeben. Die Lösung wurde auf 60 °C (Innentemperatur) erwärmt und gleichzeitig mit dem Zutropfen von 1,81 kg (22,5 mol) 2-Chlorethanol begonnen. Nach beendeter Zugabe wurde der Ansatz für 48 h bei 60 °C gerührt. Zur Aufarbeitung wurde der Ansatz anschließend mit 6,0 l Toluol verdünnt, und nach der Phasentrennung wurde die wässrige Phase noch zweimal mit je 3,0 l Toluol extrahiert. Die vereinten Toluolphasen wurden dreimal mit je 4,0 l 1N Natronlauge, dreimal mit je 4,0 l 1N Salzsäure sowie dreimal mit je 3,0 l Wasser gewaschen und das Toluol wurde bei vermindertem Druck entfernt. Nach einer Umkristallisation aus Toluol erhielt man 2,77 kg (86% Ausbeute) 2-(2-Biphenyl)-oxyethanol als farblosen, kristallinen Feststoff (Smp.: 74-75 °C) mit einer Reinheit von >99% (GC).
¹H-NMR (400 MHz, CDCl₃) *δ* (ppm) = 1,86 (t, J = 6,5 Hz, 1H, OH), 3, 78-3, 81 (m, 2H, HOC**H**₂), 4,04 (t, *J* = 4,6 Hz, 2H, OCH₂), 6,98-7,08, 7,28-7,34, 7,38-7,42 und 7,50-7,52 (4 m, 2H, 3H, 2H, 2H, =CH).
¹³C-NMR (100 MHz, CDCl₃) *δ* (ppm) = 61,4 und 70,3 (OCH₂), 113,5 (C-6), 121,7 (C-4), 127,1, 128,7 und 131,0 (C-3, C-5, C-4'), 128,1 und 129,4 (C-2', C-3', C-5', C-6'), 131,5 und 138,4 (C-2, C-1'), 155,4 (C-1).
IR (Diamant-ATR): *v* (cm⁻¹) = 3329 (br, m, OH), 3056 (m, =CH), 2918 und 2866 (m, CH₂), 1596 und 1584 (m, C=C), 1502 und 1483 (s, Aromat), 1431 (s, CH₂), 1260 und 1077 (s, COC), 1054 (s, COH), 749, 730 und 700 (vs, =CH).

### 2. Stufe: 2-(2-Biphenyloxy)-ethylmethacrylat

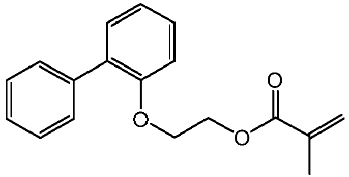

In einem Doppelmantelreaktor wurden zu einer Lösung von 1,33 kg (6,2 mol) 2-(2-Biphenyl)-oxyethanol in 13,0 l Methylenchlorid 0,75 kg (7,4 mol) Triethylamin, 37,9 g (0,31 mol) 4-Dimethylaminopyridin und 0,35 g 2,6-Di-tert-butyl-4-methylphenol zugegeben. Bei 0 °C (Innentemperatur) wurde eine Lösung von 1,14 kg (7,4 mol) Methacrylsäureanhydrid zugetropft und noch 2 h bei dieser Temperatur sowie 20 h bei 20 °C gerührt. Anschliessend wurde die Lösung dreimal mit je 4,0 l 1N Salzsäure, dreimal mit je 4,0 l 1 N Natronlauge sowie dreimal mit je 4,0 l Wasser gewaschen. Die organische Phase wurde mit 0,09 g Phenothiazin stabilisiert. Nach der Entfernung des Lösungsmittels erhielt man 1,71 kg (98% Ausbeute) 2-(2-Biphenyloxy)-ethylmethacrylat (BPOEMA) als fast farbloses Öl mit einer Reinheit von 96,45% (GC).
¹H-NMR (400 MHz, CDCl₃) *δ* (ppm) = 1,93 (s, 3H, CH₃), 4,19 und 4,41 (2 t, je *J* = 4,8 Hz, je 2H, OCH₂), 5,55 und 6,08 (2 s, je 1H, =CH₂), 6,95-7,06, 7,26-7,30, 7,33-7,37 und 7,52-7,55 (4 m, 2H, 2H, 3H, 2H, =CH).
¹³C-NMR (100 MHz, CDCl₃) *δ* (ppm) = 18,3 (CH₃), 63,0 und 66,5 (OCH₂), 113,1 (C-6), 121,7 (C-4), 126,0 (C=**C**H₂), 126,9, 128,6, 131,1 (C-3, C-5, C-4'), 127,9 und 129,6 (C-2', C-3', C-5', C-6'), 131,3 und 138,3 (C-2, C-1'), 136,1 (**C**=CH₂), 155,4 (C-1), 167,2 (C=O).
IR (Diamant-ATR): *v* (cm⁻¹) = 3027 (w, =CH), 2955 und 2900 (w, CH₂, CH₃), 1716 (vs, C=O), 1636 (m, C=C*_{Methacryl}*), 1598 und 1584 (m, C=C*_{Aromat}*), 1504 und 1482 (m, s, Aromat), 1434 (s, CH₂, CH₃), 1261 und 1125 (s, COC*_{Ether}*), 1157 (vs, COC*_{Ester}*), 939 (s, =CH*_{Methacryl}*), 751, 733 und 697 (vs, =CH*_{Aromat}*).

Die Rotationsviskosität von BPOEMA wurde mit einem MCR 302 Rheometer (Anton Paar GmbH, Österreich) mit einem Platte-Platte-Messsystem bei 23 °C zu η = 0,11 Pa·s bestimmt. Hierzu wurde die Probe vorher für 3 Minuten temperiert und die Schergeschwindigkeit zwischen 0,1 und 100 s⁻¹ gemessen. Aus dem für die Bewegung notwendigen Drehmoment wurden die Schubspannung und die Viskosität berechnet. Der Brechungsindex von BPOEMA wurde mittels eines Refraktometers ABBE 5 (Bellingham+Stanley, UK) zu n_{D}²⁰ = 1,5729 bestimmt. Die Dichte von BPOEMA wurde mittels eines Biegeschwingers Density Meter DS 7000 (Krüss, Deutschland) zu 1,119 g/cm³ bestimmt.

### Beispiel 2: Lichtinduzierte Homopolymerisation BPOEMA

Zum Monomer BPOEMA aus Beispiel 1 wurde als Photoinitiator 0,2 Gew.-% Ivocerin® (Bis(4-methoxybenzoyl)diethylgermanium, Ivoclar Vivadent AG) zugegeben. Nach Homogenisierung der Mischung wurden 10 Prüfkörper mit einem Durchmesser von 15 mm und einer Höhe von 0,5 mm präpariert und 2 mal 3 Minuten mit einer dentalen Lichtquelle (Spectramat®, Ivoclar Vivadent AG) bestrahlt und so vollständig ausgehärtet. Die Dichte der Homopolymer-Prüfkörper wurde mittels der Auftriebsmethode zu 1,209 g/cm³ bestimmt. Unter Berücksichtigung der Monomerdichte ergibt sich daraus ein Polymerisationsschrumpf von nur 7,4 Vol.-% im Vergleich zu den dentalen Verdünnermonomeren TEGDMA bzw. D₃MA, die einen Polymerisationsschrumpf von 14,5 bzw. 10,3 Vol.-% aufweisen.

### Beispiel 3: Verfolgung der Photopolymerisation von BPOEMA mittels DSC

Die Photopolymerisation von BPOEMA wurde im Vergleich zu den dentalen Verdünnermonomeren TEGDMA und D₃MA bei 30 °C Reaktionstemperatur unter Stickstoff als Schutzgas untersucht, wobei 0,5 mol-% Ivocerin® als Photoinitiator eingesetzt und als Lampe eine Bluephase (HiP) verwendet wurde. Die Ergebnisse in Figur 1 belegen eine deutlich höhere Photopolymerisationsreaktivität von BPOEMA mit einer maximalen Polymerisationsgeschwindigkeit Rₚₘₐₓ von 0,068 s⁻¹ im Vergleich zu 0,040 s⁻¹ für D₃MA und 0,050 s⁻¹ für TEGDMA.

### Beispiel 4: Kompositzement auf der Basis von BPOEMA

Aus Mischungen der Dimethacrylate UDMA (Additionsprodukt aus 2-Hydroxyethylmethacrylat und 2,2,4-Trimethylhexamethylen-1,6-diisocyanat) mit dem erfindungsgemäßen Monomer BPOEMA aus Beispiel 1 oder TEGDMA (Triethylenglycoldimethacrylat) oder Decandiol-1,10-dimethacrylat (D₃MA), dem Photoinitiatorsystem aus Campherchinon (CC) und 4-Dimethylaminobenzoesäureethylester (EDMAB) sowie den Füllstoffen silanisierte pyrogene Kieselsäure (OX-50, sil., Degussa) und YbF₃ (Ytterbiumtrifluorid, Sukgyung, Südkorea) wurden gemäß Tabelle 1 die lichthärtenden Kompositzemente **C1 bis C3** mittels eines Walzenstuhles "Exakt" (Exakt Apparatebau, Norderstedt) hergestellt.

**Tabelle 1: Zusammensetzung der Zemente C1 bis C3 (Angaben in Gew.-%)**

| Komponente | **C1** | **C2*** | **C3*** |
|---|---|---|---|
| BPOEMA¹ | 7,81 | - | - |
| UDMA² | 31,64 | 31,64 | 31,64 |
| TEGDMA³ | - | 7,81 | - |
| D₃MA⁴ | - | - | 7,81 |
| OX-50, sil.⁵ | 41,35 | 41,35 | 41,35 |
| YbF₃⁶ | 18,73 | 18,73 | 18,73 |
| CC⁷ | 0,24 | 0,24 | 0,24 |
| EDMAB⁸ | 0,23 | 0,23 | 0,23 |

| | | | |
|---|---|---|---|
| * Vergleichsbeispiel 1 = 2-(2-Biphenyloxy)-ethylmethacrylat 2 = Additionsprodukt aus 2-Hydroxyethylmethacrylat und 2,2,4-Trimethylhexamethylen-1,6-diisocyanat 3 = Triethylenglycoldimethacrylat 4 = Decandiol-1,10-dimethacrylat 5 = silanisierte pyrogene Kieselsäure, Degussa 6 = Ytterbiumtrifluorid, Sukgyung, Südkorea 7 = Campherchinon 8 = 4-Dimethylaminobenzoesäureethylester | | | |

Aus den erhaltenen Kompositzementen **C1** bis **C3** wurden Prüfkörper präpariert, die 2 mal 3 Minuten mit einer dentalen Lichtquelle (Spectramat®, Ivoclar Vivadent AG) bestrahlt und damit ausgehärtet wurden. Gemäß der ISO-Norm ISO-4049 (Dentistry - Polymer-based filling, restorative and luting materials) erfolgte die Bestimmung der Biegefestigkeit (BF) und des Biege-E-Moduls (BM) nach 24 h Lagerung der Prüfkörper in Wasser (WL) bei 37 °C (Tabelle 2).

**Tabelle 2: Biegefestigkeit (BF, MPa) und Biege-E-Modul (BM, GPa) der polymerisierten Zemente C1 bis C3**

| | **C1** | **C2*** | **C3*** |
|---|---|---|---|
| BF, WL | 126,2±7,2 | 127,2±11,8 | 118,6±11,8 |
| BM, WL | 5,64±0,16 | 5,64±0,50 | 5,04±0,36 |

| | | | |
|---|---|---|---|
| * Vergleichsbeispiel | | | |

Das Beispiel 4 zeigt, dass die Verwendung von erfindungsgemäßen gering schrumpfenden Biphenylmethacrylaten anstatt bekannter dentaler Dimethacrylatverdünnermonomere Kompositzemente mit vergleichbaren mechanischen Eigenschaften ergibt.

### Beispiel 5: Fliessfähiges Füllungskomposit auf der Basis von BPOEMA

Aus Mischungen der Dimethacrylate UDMA und Bis-GMA (Additionsprodukt aus Methacrylsäure und Bisphenol-A-diglycidylether) mit dem erfindungsgemässen Monomer BPOEMA aus Beispiel 1 oder TEGDMA oder D₃MA, dem Photoinitiatorsystem aus Campherchinon (CC) und 4-Dimethylaminobenzoesäureethylester (EDMAB) sowie den Füllstoffen silanisierte pyrogene Kieselsäure (OX-50, sil., Degussa), YbF₃, silanisiertes SiO₂-ZrO₂-Mischoxid, (Transparent Materials, USA) und silanisiertes Bariumaluminiumsilikatglas (GM 27884, mittlere Partikelgröße 1,0 µm, Schott) wurden gemäß Tabelle 3 die lichthärtenden, fliessfähigen Füllungskomposite **K1-K3** mittels eines Walzenstuhles hergestellt.

**Tabelle 3: Zusammensetzung der Füllungskomposite K1 bis K3 (Angaben in Gew.-%)**

| Komponente | **K1** | **K2*** | **K3*** |
|---|---|---|---|
| BPOEMA¹ | 10,09 | - | - |
| UDMA² | 9,15 | 9,15 | 9,15 |
| BisGMA³ | 13,98 | 13,98 | 13,98 |
| TEGDMA⁴ | - | 10,09 | - |
| D₃MA⁵ | - | - | 10,09 |
| OX-50, sil.⁶ | 3,62 | 3,62 | 3,62 |
| YbF₃⁷ | 2,51 | 2,51 | 2,51 |
| SiOᵣZrO₂ sil.⁸ | 4,02 | 4,02 | 4,02 |
| GM 27884 sil.⁹ | 56,22 | 56,22 | 56,22 |
| CC¹⁰ | 0,21 | 0,21 | 0,21 |
| EDMAB¹¹ | 0,20 | 0,20 | 0,20 |

| | | | |
|---|---|---|---|
| * Vergleichsbeispiel 1 = 2-(2-Biphenyloxy)-ethylmethacrylat 2 = Additionsprodukt aus 2-Hydroxyethylmethacrylat und 2,2,4-Trimethylhexamethylen-1,6-diisocyanat 3 = Additionsprodukt aus Methacrylsäure und Bisphenol-A-diglycidylether 4 = Triethylenglycoldimethacrylat 5 = Decandiol-1,10-dimethacrylat 6 = silanisierte pyrogene Kieselsäure, Degussa 7 = Ytterbiumtrifluorid, Sukgyung, Südkorea 8 = silanisiertes SiO₂-ZrO₂-Mischoxid (Transparent Materials, USA) 9 = silanisiertes Bariumaluminiumsilikatglas (GM 27884, mittlere Partikelgröße 1,0 µm, Schott) 10 = Campherchinon 11 = 4-Dimethylaminobenzoesäureethylester | | | |

Aus den erhaltenen Füllungskompositen wurden Prüfkörper präpariert, die 2 mal 3 Minuten mit einer dentalen Lichtquelle (Spectramat®) bestrahlt und damit ausgehärtet wurden. Die Bestimmung der Biegefestigkeit (BF) und des Biege-E-Moduls (BM) erfolgte nach 7 Tage Lagerung der Prüfkörper in Wasser (WL) bei 37 °C (Tabelle 4).

**Tabelle 4: Biegefestigkeit (BF, MPa) und Biege-E-Modul (BM, GPa) der polymerisierten Komposite K1 bis K3**

| | **K1** | **K2*** | **K3*** |
|---|---|---|---|
| BF, WL | 125,4±12,4 | 139,6±10,4 | 127,0±10,8 |
| BM, WL | 7,74±0,28 | 7,74±0,59 | 6,74±0,20 |

| | | | |
|---|---|---|---|
| * Vergleichsbeispiel | | | |

Das Beispiel 5 zeigt, dass die Verwendung von erfindungsgemäßen gering schrumpfenden Biphenylmethacrylaten anstatt bekannter dentaler Dimethacrylatverdünnermonomere Füllungskomposite mit vergleichbaren mechanischen Eigenschaften ergibt.

## Patentansprüche

1. Radikalisch polymerisierbarer Dentalwerkstoff, der mindestens ein radikalisch polymerisierbares, monofunktionelles Biphenylmethacrylat gemäß der Formel I enthält, 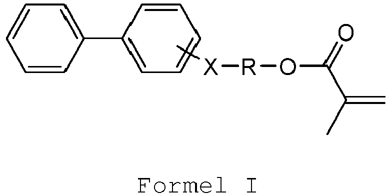 in der
R ein linearer oder verzweigter C₁-C₁₄-Alkylrest ist, der durch O oder S unterbrochen sein kann,
X entfällt oder -O-, -CO-O- oder -O-CO- ist
und die Substitution am Aromaten an Position 2, 3 oder 4 erfolgt.

2. Dentalwerkstoff nach Anspruch 1, wobei die Variablen die folgenden Bedeutungen haben:
R verzweigter oder vorzugsweise linearer C₁-C₈-Alkylrest, insbesondere ein C₂-C₆-Alkylrest, der durch O oder S unterbrochen sein kann,
X O
und die Substitution an Position 2 erfolgt.

3. Dentalwerkstoff nach Anspruch 1, wobei die Variablen die folgenden Bedeutungen haben:
R verzweigter oder vorzugsweise linearer C₁-C₈-Alkylrest, insbesondere ein C₂-C₆-Alkylrest, der durch O oder S unterbrochen sein kann,
X O
und die Substitution an Position 3 erfolgt.

4. Dentalwerkstoff nach Anspruch 1, wobei die Variablen die folgenden Bedeutungen haben:
R verzweigter oder vorzugsweise linearer C₁-C₈-Alkylrest, insbesondere ein C₂-C₆-Alkylrest, der durch 0 oder S unterbrochen sein kann,
X -O-CO- oder -CO-O-
und die Substitution an Position 2 erfolgt.

5. Dentalwerkstoff nach Anspruch 1 oder 2, wobei die Variablen die folgenden Bedeutungen haben:
R linearer C₂- oder C₄-Alkylrest,
X O
und die Substitution an Position 2 erfolgt.

6. Dentalwerkstoff nach einem der Ansprüche 1 bis 5, der zusätzlich mindestens einen Initiator für die radikalische Polymerisation, vorzugsweise einen Photoinitiator enthält.

7. Dentalwerkstoff nach einem der Ansprüche 1 bis 6, der zusätzlich mindestens ein weiteres radikalisch polymerisierbares Monomer, vorzugsweise mindestens ein mono- oder multifunktionelles (Meth)acrylat, besonders bevorzugt ein Dimethacrylat enthält.

8. Dentalwerkstoff nach Anspruch 7, der als zusätzliches Monomer 2,2-Bis[4-(2-hydroxy-3-methacryloyloxypropyl)phenyl]-propan (Bis-GMA), 1,6-Bis-[2-methacryloyloxyethoxycarbonyl-amino]-2,2,4-trimethylhexan (UDMA), Decandiol-1,10-dimethacrylat (D₃MA), Triethylenglycoldimethacrylat (TEGDMA), 2-[4-(2-Methacryloyloxyethoxyethoxy)phenyl]-2-[4-(2-methacryloyloxyethoxy)phenyl]-propan) (SR-348c) oder eine Mischung davon enthält.

9. Dentalwerkstoff nach einem der Ansprüche 1 bis 8, der zusätzlich mindestens einen anorganischen Füllstoff enthält.

10. Dentalwerkstoff nach Anspruch 9, der als anorganischen Füllstoff pyrogene Kieselsäure, Ytterbiumfluorid, SiO₂-ZrO₂-Mischoxide, Bariumaluminiumsilikatgläser oder Mischungen davon enthält.

11. Dentalwerkstoff nach einem der Ansprüche 1 bis 10, der
(a) 0,5 bis 60 Gew.-%, bevorzugt 1 bis 50 Gew.-% und besonders bevorzugt 3 bis 30 Gew.-% mindestens eines monofunktionellen Biphenylmethacrylats gemäß der Formel I,
(b) 0,01 bis 5 Gew.-%, besonders bevorzugt 0,1 bis 3,0 Gew.-% mindestens eines Initiators für die radikalische Polymerisation und
(c) 5 bis 70 Gew.-%, bevorzugt 10 bis 60 Gew.-% und besonders bevorzugt 10 bis 50 Gew.-% mindestens eines weiteren radikalisch polymerisierbaren Monomers enthält, jeweils bezogen auf die Gesamtmasse des Werkstoffs.

12. Dentalwerkstoff nach Anspruch 11, der zusätzlich
(d) 0 bis 80 Gew.-%, bevorzugt 10-70 Gew.-% oder 50-80 Gew.-% mindestens eines Füllstoffs enthält, jeweils bezogen auf die Gesamtmasse des Werkstoffs.

13. Dentalwerkstoff nach einem der Ansprüche 1 bis 12 zur therapeutischen Anwendung als dentaler Zement, Füllungskomposit oder Verblendmaterial.

14. Verwendung eines Dentalwerkstoffs gemäß einem der Ansprüche 1 bis 12 zur extraoralen Herstellung oder Reparatur von Dentalrestaurationen, Prothesen, künstlichen Zähnen, Inlays, Onlays, Kronen oder Brücken.

15. Verwendung eines monofunktionellen Biphenylmethacrylats gemäß Formel I zur Herstellung eines Dentalwerkstoffs.
